Europäisches Patentamt

European Patent Office

Office européen des brevets

(11) Veröffentlichungsnummer: **0 303 985**

**A2**

(12) **EUROPÄISCHE PATENTANMELDUNG**

(21) Anmeldenummer: 88113126.2

(51) Int. Cl.4: **A61J 1/00 , A61M 5/14**

(22) Anmeldetag: **12.08.88**

(30) Priorität: **19.08.87 DE 3727627**

(43) Veröffentlichungstag der Anmeldung:
**22.02.89 Patentblatt 89/08**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Schwabe, Karl-Detlev, Dr.**
**Mailänder Strasse 16/163**
**D-6000 Frankfurt am Main(DE)**
Erfinder: **Pohler, Wolfgang, Dr.**
**Martin-Wohmann-Strasse 27**
**D-6238 Hofheim am Taunus(DE)**
Erfinder: **Toth, Elisabeth**
**Kölner Strasse 46**
**D-6090 Rüsselsheim(DE)**

(54) **Verwendung von Folien aus Kunststoffen mit polaren Molekülgruppen als Primärpackmittel für Beta-Laktam-Antibiotika sowie Kunststoffbeutel.**

(57) Es wird die Verwendung von Folien aus Kunststoffen mit polaren Molekülgruppen als Primärpackmittel für feste $\beta$-Laktam-Antibiotika beschrieben. Primärpackmittel sind Kunststoffbeutel aus den genannten Kunststoffen.

EP 0 303 985 A2

# Verwendung von Folien aus Kunststoffen mit polaren Molekülgruppen als Primärpackmittel für β-Laktam-Antibiotika sowie Kunststoffbeutel.

Aseptisch zu handhabende sterile Cephalosporinderivate. die zur Herstellung von Injektionszubereitungen verwendet werden sollen. wie z.B. Cefotaxim, Cefodizim, Cefpirom, oder auch 1-[[(6R,7R)-7-[2-(2-Amino-4-thiazolyl)glyoxylamido]-2-carboxy-8-oxo-5-thia-1-azabicyclo[4.2.0]-oct-2-en-3-yl]methyl]-5,6,7,8-tetrahydrochinoliniumhydroxid, inneres Salz $7^2$-(Z)-(O-methyloxim) (auch als HOE 111 bezeichnet) sowie deren physiologisch verträgliche Salze, gegebenenfalls im Gemisch mit pH-regulierenden Substanzen, werden in sterilen Polyethylen-Kunststoffbeuteln als Primärpackmittel verpackt. Die flexiblen Beutel sind einfach zu handhaben und zeigen keinen Partikelabrieb. wie er insbesondere an den Verschlußelementen bei nicht flexiblen Primärpackmitteln, z.B. aus Edelstahl oder Glas, auftritt.

Nachteilig ist, daß Polyethylen- und in gleicher Weise auch Polypropylen-Kunststoffbeutel bei der Lagerung zu Trüblöslichkeit der verpackten Substanzen führen, selbst wenn sowohl Beutel als auch abzupackende Substanz steril waren und das Abpacken unter aseptischen Bedingungen erfolgte. Substanzen, die zur Herstellung von Injektionslösungen verwendet werden, müssen jedoch klarlöslich sein.

Versuche, die bekannten Primärpackmittel zu ersetzen durch solche aus Verbundfolie aus Polyamid/Hochdruckpolyethylen, Verbundfolie aus Polyester/Hochdruckpolyethylen oder Verbundfolie aus Polyester/Aluminium/Polyethylen wie sie vorwiegend für die Verpackung von Lebensmitteln verwendet werden, zeigten nicht den gewünschten Erfolg; es wurde eine im gleichen Maße auftretende Trüblöslichkeit festgestellt. Sie stellen keinerlei Verbesserung zu den verwendeten Polyethylen- und Polypropylenbeuteln dar. Aufgrund dieser Beobachtungen mußte der Fachmann erwarten, daß auch Beutel aus Kunststoffen mit polaren Molekülgruppen als Primärpackmittel für β-Laktam-Antibiotika ungeeignet sind, da sie chemisch reaktiver sind als Polyethylen und Polypropylen.

Es wurde nun überraschenderweise gefunden, daß bei der Verwendung von Kunststoffbeuteln aus Kunststoffen mit polaren Molekülgruppen wie z.B. Zellglasfolie, Polyamid, Polyester, Polyurethan oder Zelluloseacetat, keine Trüblöslichkeit bei Cephalosporinderivaten hervorgerufen wird.

Die Erfindung betrifft daher die Verwendung von Folien aus Kunststoffen mit polaren Molekülgruppen als Primärpackmittel für feste β-Laktam-Antibiotika sowie Kunststoffbeutel aus Folien von Kunststoffen mit polaren Molekülgruppen als Primärpackmittel für feste β-Laktam-Antibiotika.

Als Kunststoffe mit polaren Molekülgruppen kommen insbesondere Zellglas (z.B. ®Cellophan), Zelluloseacetat, Polyamid (z.B. ®Pla-Steril), Polyester und Polyurethane in Betracht. Vorzugsweise werden Zellglas, Polyamid und Polyester verwendet.

Kunststoffbeutel aus den genannten Folien können im Gegensatz zu Kunststoffbeuteln aus Polyethylen im Autoklaven sterilisiert werden. Eine sterilisierende Begasung, z.B. mit Formaldehyd oder Ethylenoxid ist möglich, kann aber wegen der Wärmebeständigkeit vermieden werden, was den Vorteil hat, daß Rückstände des sterilisierenden Gases in dem Primärpackmittel nicht mehr auftreten können.

Die Folien sind unter aseptischen Bedingungen oder Reinraumbedingungen herstellbar und dürfen mit keinerlei Staubteilchen - auch sterilen - behaftet sein, d.h. eine Kontamination des Arzneistoffes muß von vorneherein ausgeschlossen sein.

Die Beutel erhält man z.B. durch Falten oder Schweißen der Folie. Die Beutel für die Primärverpackung werden mit Kabelbindern versiegelt. Die Dicke der Folie richtet sich selbstverständlich nach der Festigkeit der Folie und nach der Menge des im Beutel abzufüllenden Wirkstoffs. Die Beutel besitzen ausreichende mechanische Stabilität, ein Abrieb wurde nicht beobachtet.

Als β-Laktam-Antibiotika kommen in erster Linie Cephalosporine in Betracht, insbesondere Cefotaxim, Cefodizim, Cefpirom und HOE 111, sowie deren physiologisch verträgliche Salze, gegebenenfalls im Gemisch mit pH-regulierenden Verbindungen. Als pH-regulierende Verbindungen kommen z.B. Natriumcarbonat, Trinatriumphosphat und basische Aminosäuren in Frage.

Die nachstehenden Beispiele zeigen, daß bei Verwendung von Folien aus den genannten Kunststoffen die für Arzneistoffe vorgeschriebene Klarlöslichkeit auch unter verschiedenen Arten der Belastung, beobachtet wurde, während bei der Verwendung von Polyethylenfolie bereits mit dem Auge eine Trüblöslichkeit wahrnehmbar war.

Beispiel I

| Cefodizim-di-Na-Salz 0,5 g Dosierung verpackt in Beuteln (5 cm Länge, 4 cm Breite) | | | | |
|---|---|---|---|---|
| | Polyethylenfolie | Polyamidfolie | Zellglasfolie | Polyesterfolie |
| 1 h Vibration | trüblöslich | klarlöslich | klarlöslich | klarlöslich |
| 1 h Vibration danach 7 Tage Lagerung bei + 40°C | trüblöslich | klarlöslich | klarlöslich | klarlöslich |
| 1 h Vibration, danach 7 Tage Lagerung bei Raumtemperatur | trüblöslich | klarlöslich | klarlöslich | klarlöslich |
| 1 h Vibration, danach 7 Tage Lagerung bei + 5°C | trüblöslich | klarlöslich | klarlöslich | klarlöslich |
| 7 Tage Lagerung bei + 40°C | trüblöslich | klarlöslich | klarlöslich | klarlöslich |
| 7 Tage Lagerung bei Raumtemperatur | trüblöslich | klarlöslich | klarlöslich | klarlöslich |
| 7 Tage Lagerung bei + 5°C | trüblöslich | klarlöslich | klarlöslich | klarlöslich |

Beispiel II

| Cefotaxim-Na-Salz 0,5 g Dosierung verpackt in Beuteln (5 cm Länge, 4 cm Breite) | | | | |
|---|---|---|---|---|
| | Polyethylenfolie | Polyamidfolie | Zellglasfolie | Polyesterfolie |
| 1 h Vibration | trüblöslich | klarlöslich | klarlöslich | klarlöslich |
| 1 h Vibration, danach 7 Tage Lagerung bei + 40°C | trüblöslich | klarlöslich | klarlöslich | klarlöslich |
| 1 h Vibration, danach 7 Tage Lagerung bei Raumtemperatur | trüblöslich | klarlöslich | klarlöslich | klarlöslich |
| 1 h Vibration, danach 7 Tage Lagerung bei + 5°C | trüblöslich | klarlöslich | klarlöslich | klarlöslich |
| 7 Tage Lagerung bei + 40°C | trüblöslich | klarlöslich | klarlöslich | klarlöslich |
| 7 Tage Lagerung bei Raumtemperatur | trüblöslich | klarlöslich | klarlöslich | klarlöslich |
| 7 Tage Lagerung bei + 5°C | trüblöslich | klarlöslich | klarlöslich | klarlöslich |

Beispiel III

| Cefpiromsulfat - Natriumkarbonatgemisch mit 5 cm² Beutelfolie pro g | | | | | |
|---|---|---|---|---|---|
| | Polyethylenfolie | Polyamidfolie | Cellophanfolie | Polyesterfolie | ohne Folie als Vergleich |
| 7 Tage rollen | trüblöslich | klarlöslich | klarlöslich | klarlöslich | klarlöslich |
| 7 Tage Lagerung bei Raumtemperatur nach Durchmischung | trüblöslich | klarlöslich | klarlöslich | klarlöslich | klarlöslich |
| 7 Tage Lagerung bei + 40°C nach Durchmischung | trüblöslich | klarlöslich | klarlöslich | klarlöslich | klarlöslich |

**Ansprüche**

1. Verwendung von Folien aus Kunststoffen mit polaren Molekülgruppen als Primärpackmittel für feste β-Laktam-Antibiotika.

2. Kunststoffbeutel aus Folien von Kunststoffen mit polaren Molekülgruppen als Primärpackmittel für feste β-Laktam-Antibiotika.

3. Kunststoffbeutel aus Folien von Kunststoffen mit polaren Molekülgruppen als Primärpackmittel für feste Cephalosporinderivate.

4. Kunststoffbeutel aus Folien von Kunststoffen mit polaren Molekülgruppen als Primärpackmittel für Cefotaxim, Cefodizim, Cefpirom, und HOE 111, sowie für deren physiologisch verträgliche Salze, gegebenenfalls im Gemisch mit pH-regulierenden Verbindungen, in fester Form.

5. Verwendung von Folien aus Zellglas, Zelluloseacetat, Polyamid, Polyester oder Polyurethan als Primärpackmittel für feste β-Laktam-Antibiotika.

6. Kunststoffbeutel aus Folien von Zellglas, Zelluloseacetat, Polyamid, Polyester oder Polyurethan als Primärpackmittel für feste β-Laktam-Antibiotika.

7. Verfahren zur Verpackung von festen β-Laktam-Antibiotika, dadurch gekennzeichnet, daß man als Primärpackmittel Kunststoffbeutel aus Folien von Kunststoffen mit polaren Molekülgruppen verwendet.